# FASCICULE DE BREVET EUROPEEN

(11) **EP 0 601 918 B1**
(45) Date de publication et mention de la délivrance du brevet: **18.03.1998**
(21) Numéro de dépôt: 93402930.7
(22) Date de dépôt: 03.12.1993
(51) Int. Cl.: C07D 317/26, C07D 317/16, C07D 319/06, C07C 403/14

(54) **Procédé de préparation de la vitamine A et composés intermédiaires utiles pour ce procédé**
Verfahren zur Herstellung von Vitamine A und Zwischenprodukte dafür
Process for the preparation of vitamin A and intermediates useful in this process

(30) Priorité: 07.12.1992 FR 9214697
(43) Date de publication de la demande: 15.06.1994
(73) Titulaire: RHONE-POULENC NUTRITION ANIMALE, 92160 Antony (FR)
(72) Inventeur: Chabardes, Pierre, F-69110 Sainte Foy les Lyon (FR)
(74) Mandataire: Le Pennec, Magali

(56) Documents cités:
- EP-A- 0 145 554
- EP-A- 0 187 259

## Description

La présente invention concerne un nouveau procédé de préparation de la vitamine A, elle concerne également de nouveaux intermédiaires utilisés pour cette synthèse.

Il est connu selon la publication parue dans le Journal of American Chemical Society, 106, 3670 (1984) de préparer la vitamine A par condensation de la cyclogéranylsulfone avec l'ester méthylique de l'acide formyl-7 méthyl-3 octadiène 2,6 selon le schéma réactionnel suivant :

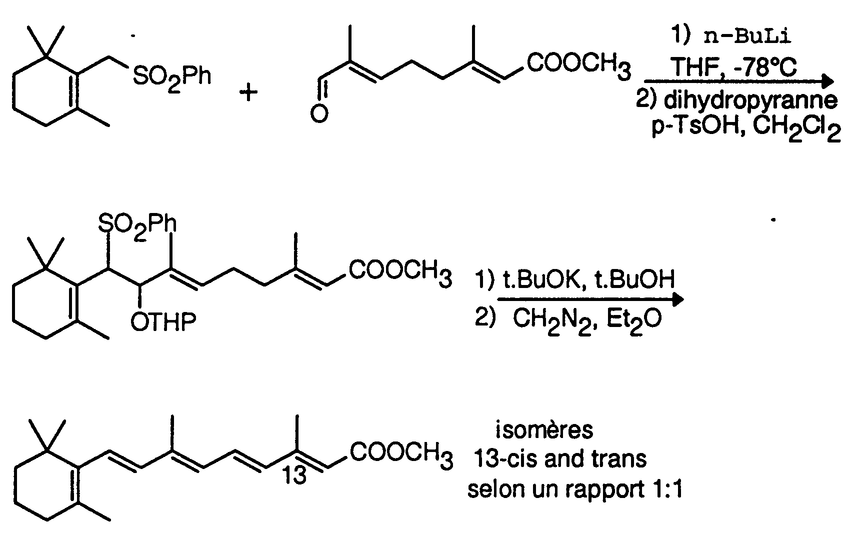

Selon cette publication, il était uniquement possible d'obtenir l'ester méthylique de l'acide rétinoïque selon un rapport isomérique cis/trans, sur la liaison 13, de 1/1.

Pour s'affranchir de cet inconvénient, l'auteur de cette publication a réalisé la même condensation à partir de l'alcool correspondant en C₁₀ à la place de l'ester méthylique de l'acide insaturé en C₁₀ ou à partir de l'acétate de ce même alcool en utilisant comme milieu de condensation un solvant hydrocarboné aliphatique ou aromatique.

Ce nouveau procédé est décrit notamment par OTERA et Coll. dans le Journal of Organic Chemistry, 1986, 51, page 3834.

Le rendement obtenu par ce procédé en acétates de vitamine A atteint environ 60 %, mais demande un isolement intermédiaire à chaque étape, ce qui est coûteux dans un procédé industriel. Ce procédé exige aussi de partir de l'intermédiaire en C₁₀ dans lequel les liaisons en position 2 et 6 sont entièrement trans car il n'y a aucune étape de redressement dans ce procédé.

Il est d'ailleurs précisé dans le texte du brevet EP 187 259, correspondant à la dernière publication mentionnée préalablement, à la page 19 que la stéréochimie de la vitamine A dépend de la stéréochimie du composé de départ c'est-à-dire de l'aldéhyde alcool en C₁₀ représenté par la formule suivante :

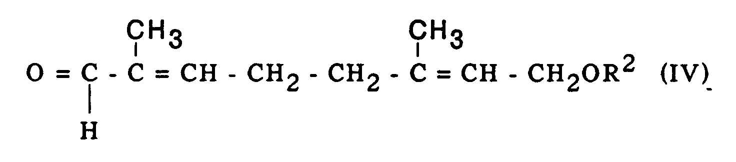

Il est précisé que si la stéréochimie du composé de formule (IV) est restreinte à des composés ayant uniquement des liaisons 2 et 6 tout trans, la stéréochimie de la vitamine A obtenue sera alors restreinte à une stéréochimie tout trans.

Le procédé de préparation de la vitamine A selon ce brevet et cette publication est donc restreint, comme matière première insaturée en C₁₀, à l'utilisation de l'acétate de géranyle qui est une matière première onéreuse car souvent accompagné de son isomère 2 cis qui est l'acétate de néryle. Il est toujours difficile d'obtenir des doubles liaisons trisubstituées de stéréochimie (cis ou trans).

Le procédé de la présente invention consiste à condenser la cyclogéranylsulfone avec un aldéhyde acétal en C₁₀ de formule suivante : 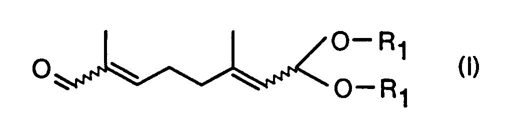 dans laquelle chaque motif R₁ représente un motif alkyle contenant de préférence 1 à 6 atomes de carbone ou forme ensemble un motif alkylidène contenant de préférence 2 à 6 atomes de carbone.

Les composés de formule (I) préférés sont représentés par un mélange des quatre isomères suivants :
acétal-8 du diméthyl 2,6 octadiène -2(E), 6(E) dial-1,8
acétal-8 du diméthyl 2,6 octadiène -2(Z), 6(E) dial-1,8
acétal-8 du diméthyl 2,6 octadiène - 2(E), 6(Z) dial,1,8
acétal-8 du diméthyl 2,6 octadiène - 2(Z), 6(Z) dial,1,8

Les composés de formule (I) sont des composés nouveaux. Ces composés de formule (I) sont facilement obtenus par oxydation d'un halogénoacétal de formule (II) suivante au moyen d'un N-oxyde d'une amine tertiaire selon le schéma suivant :

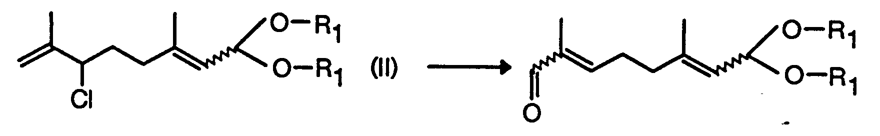

On préfère utiliser comme oxydant le N-oxyde de la méthyl-4 morpholine et le N-oxyde de la triméthylamine.

Les composés de formule (II) sont obtenus à partir du citral par réaction successive d'acétalisation et d'halogénation.

Ainsi, selon un premier procédé de préparation de (II), on met en présence le citral avec un agent d'halogénation choisi notamment parmi l'hypochlorite de sodium ou directement par le chlore gazeux en milieu tamponné selon le schéma réactionnel suivant :

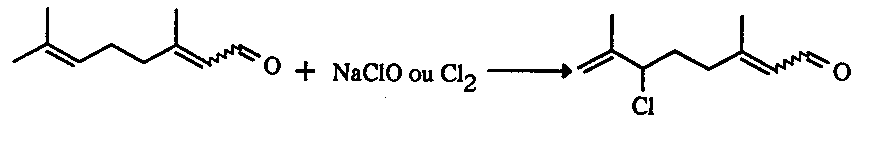

On obtient un mélange des isomères du chloro-3 diméthyl-2,6 octa dien-1,6 al-8.

Puis on réalise une acétalisation du chlorocitral. Cette acétalisation est réalisée notamment au moyen d'un glycol, de propane diol-1,3 ou de butanediol-1,4 en présence d'un sel d'ammonium tel que par exemple le para toluène sulfonate de pyridinium et d'un orthoformiate de trialkyle tel que le triméthyl orthoformiate. Cette méthode est décrite par HEGDE et Coll. dans Tetrahedron Letters, 21, 441 (1980).

Le deuxième procédé de préparation des composés de formule (II) consiste à réaliser d'abord l'acétalisation du citral puis la chloration de l'acétal obtenu dans les mêmes conditions que précédemment..

Le procédé de préparation de la vitamine A consiste :
. dans une première étape à condenser la βcyclogéranylsulfone avec les composés de formule (I) selon le schéma suivant : 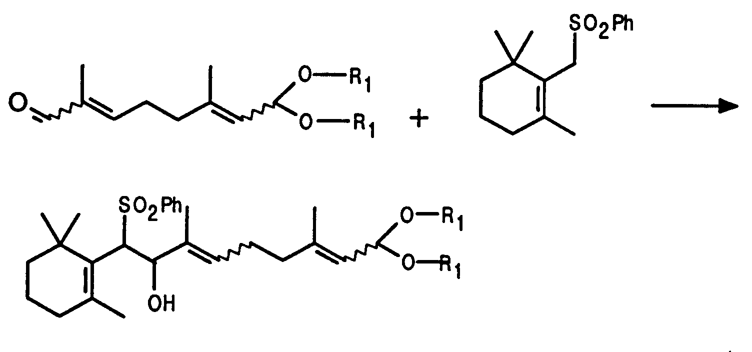
. dans une deuxième étape, on réalise une halogénation du composé obtenu à l'étape (1) selon le schéma suivant : 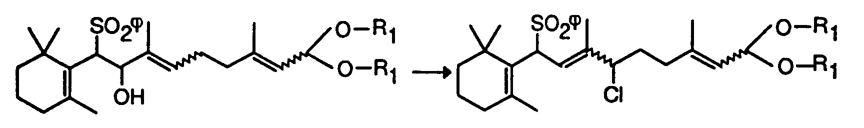
. dans une troisième étape, on réalise une double élimination de l'halogène et de la sulfone 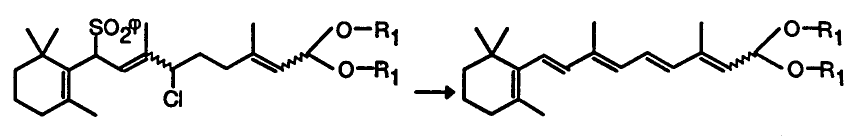
. dans une quatrième étape, on hydrolyse l'acétal 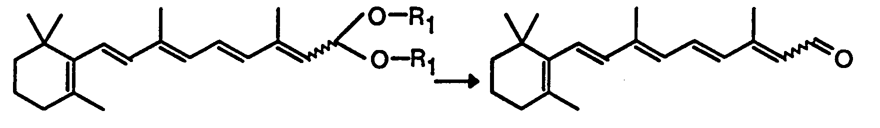
. dans une cinquième étape, on réalise un redressement du rétinal.

La première étape est réalisée de préférence en présence d'une base capable de générer un carbanion sur la cyclogéranyl sulfone. On peut utiliser comme base les organolithiens tel que le butyllithium ou les organomagnésiens tels que le chlorure de méthylmagnésium, le bromure de méthylmagnésium, les halogénures d'éthyl et propyl magnésium. On peut aussi utiliser comme base les hydrures alcalins, les amides alcalins, les alcoolates alcalins (méthylate de sodium et éthylate de sodium).

Le solvant de la première étape est, de préférence un solvant hydrocarboné choisi parmi les alcanes, les éthers, les solvants aromatiques, le solvants chlorés.

La deuxième étape est réalisée en présence de chlorure de thionyle ou de trichlorure de phosphore dans un solvant organique en présence d'une amine tertiaire.

La troisième étape est réalisée en présence d'un alcoolate alcalin dans un solvant aprotique apolaire tel qu'un hydrocarbure par exemple le toluène, le cyclohexane, l'hexane.

Les étapes 1 à 3 sont réalisées conformément au brevet EP 187 259 sans se préoccuper de la répartition des isomères sur la chaîne de la vitamine A.

L'étape 4 consiste à éliminer le groupe acétal de façon à libérer le rétinal. Cette étape est réalisée en présence d'un acide doux sous forme d'ammonium quaternaire tel que par exemple le p.toluènesulfonate de pyridinium et d'eau.

Le rétinal obtenu est un mélange d'isomères cis et trans sur la double liaison 13.

Il est redressé en rétinal tout trans à l'aide d'un complexe avec le dihydroxybenzène tel que par exemple décrit dans le brevet US 2 683 746, US 2 693 747 ou FR 1291622.

On préfère effectuer le redressement en présence d'hydroquinone et d'iode.

Par le procédé de la présente invention, il est possible dans le même réacteur, à partir de l'aldéhyde acétal en C₁₀ de formule (I) d'obtenir un mélange d'isomère 13 cis/13 trans de rétinal sans isoler aucun intermédiaire, ce qui est un énorme avantage d'un point de vue industriel.

La présente invention sera plus complètement décrite à l'aide des exemples suivants qui ne doivent pas être considérés comme limitatifs de l'invention.

### EXEMPLE 1

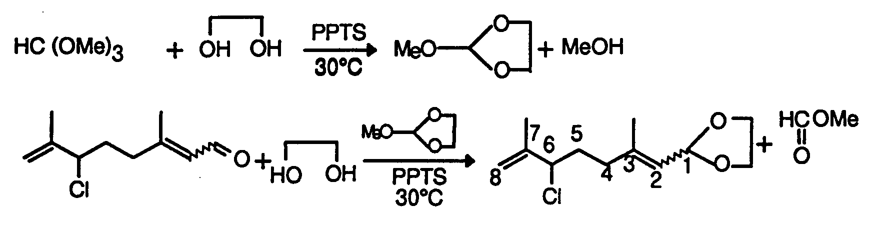

Dans un ballon de 25 ml, muni d'une agitation et d'une allonge de distillation et d'un récepteur de 5 ml, on charge :
- 0,067 g de toluènesulfonate de pyridinium à 98 %
- 0,698 g d'orthoformiate de triméthyle à 97 %
- 4 ml d'éthylèneglycol

On agite, on met l'installation sous vide ( environ 100 mbar) et on chauffe à 30°C pendant 1 heure 30. On coule ensuite goutte à goutte 1,02 g de chlorocitral à 95 % à l'aide d'une seringue que l'on rince ensuite avec 0,5 ml d'éthylèneglycol. On agite 3 heures à 30°C, puis refroidit avant d'extraire au pentane. La phase organique est lavée plusieurs fois avec une solution saturée de bicarbonate de sodium (pH = 8-9) puis avec une solution saturée de chlorure de sodium (pH = 6-7). La phase organique est séchée sur sulfate de magnésium et concentrée. Le liquide jaune vif résiduel est purifié par chromatographie préparative (phase : Flonsil φ 0,075 - 0,150 mm, Eluant : pentane 100 ml/pentane-9 éther-1 200 ml/pentane-8 éther-2 100 ml). On obtient 0,507 g d'un liquide incolore identifié en RMN comme étant la structure attendue (2 isomères). Rendement : 40 %.

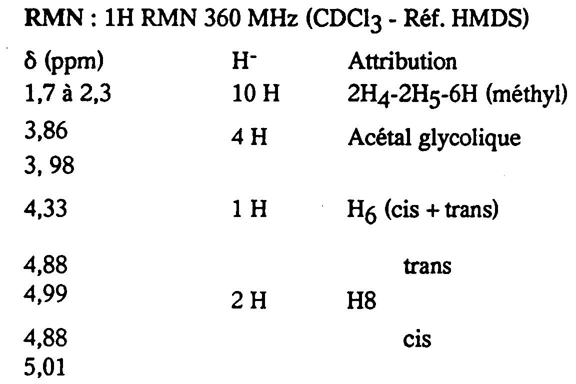

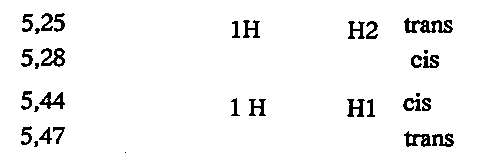

### EXEMPLE 2

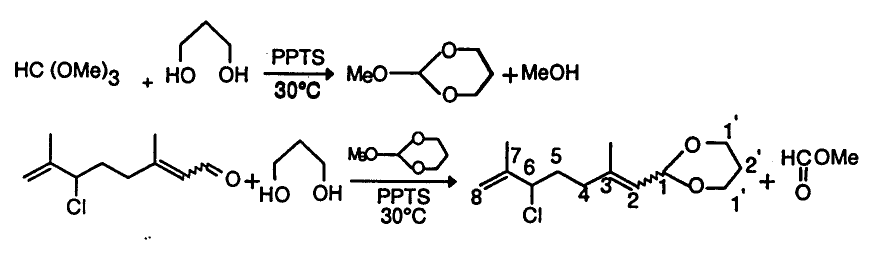

Dans un tétracol de 250 ml, muni d'une agitation, d'un thermomètre, d'une ampoule de coulée 25 ml, d'un bloc allongé de distillation, d'un réfrigérant, d'un récepteur 100 ml, on charge :
- 0,758 g de p.toluènesulfonate de pyridinium à 98%
- 55 ml de propanediol-1,3
- 9 ml d'orthoformiate de triméthyle à 97 %.

On agite, on met l'installation sous vide (environ 100 mbar) et on chauffe à 30°C pendant 1 heure 30.

On coupe le vide et on installe une circulation d'argon. On additionne lentement 10,964 g de chlorocitral à 95 % dilué dans 7 ml de THF anhydre (durée 10 mn). On rince l'ampoule de coulée avec 3 ml de THF et on laisse agiter à 30°C pendant 2 heures 30. On refroidit à 20°C, on extrait avec un mélange éther-1 pentane-10 et on lave avec successivement une solution saturée de bicarbonate de sodium et une solution saturée de chlorure de sodium. La phase organique est séchée sur sulfate de magnésium et concentrée. La liquide jaune obtenu est passé sur colonne préparative (phase Florisil φ 0,075 - 0,150 mm. Eluant : gradient pentane ---- pentane-1 éther-1).

On obtient 10,99 g de chloroacétal identifié par RMN. Rendement : 80 % (isomères trans : 67% - cis : 33 %)

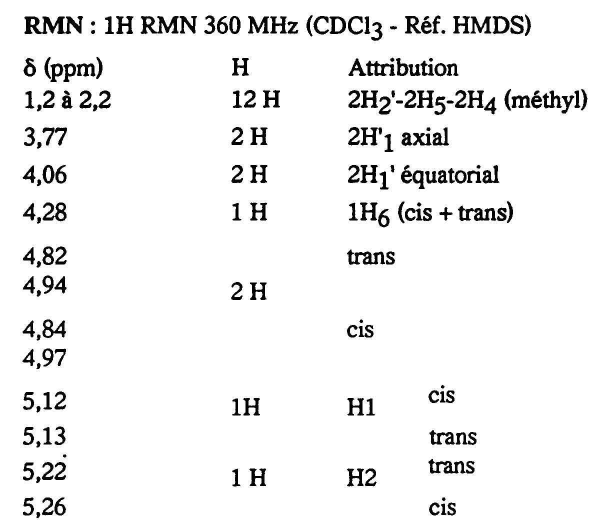

### EXEMPLE 3

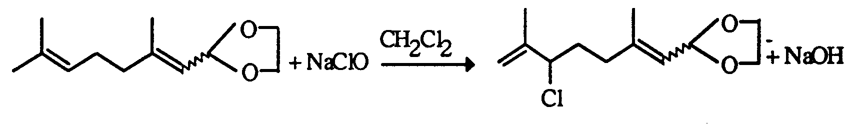

Dans un tricol de 500 ml muni d'une agitation, d'un thermomètre, d'une sonde de pH-métrie et d'une ampoule de coulée, on charge
- 12,84 g d'acétalglycolique du citral
- 160 ml de dichlorométhane.

On refroidit le milieu dans un bain de glace. On ajoute lentement 89 ml d'eau de javel (à 0,94 mol.1⁻¹). Le pH est maintenu entre 7 et 8 par addition de carboglace et la température reste comprise entre 0 et 10°C. On laisse agiter pendant 3 heures à cette température. On ajoute alors 150 ml d'eau, on extrait plusieurs fois avec du dichlorométhane. La phase organique est ensuite lavée successivement avec une solution de sulfite de sodium à 10 % et une solution saturée de bicarbonate de sodium.

Après séchage sur MgSO₄ et concentration, on obtient 14,73 g d'un liquide jaune pâle analysé en CPG. Le rendement en chloroacétal est d'environ 80 % (calculé d'après les rapports de surfaces en CPG). La RMN est identique à celle de l'exemple 1

### EXEMPLE 4

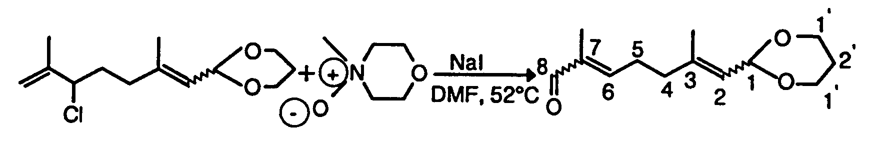

Dans un tétracol de 250 ml muni d'une agitation, d'un thermomètre et balayé à l'argon, on charge :
- 6,021 g de chloroacétal à 90 %
- 40 ml de DMF anhydre
- 4,042 g d'iodure de sodium
- 7,51 g de -4 méthylmorpholine N-oxyde à 97% anhydre.

On agite pendant 5 heures à 52°C. On plonge le ballon dans un bain de glace avant d'ajouter de l'éther et une solution saturée de bicarbonate de sodium.

On extrait à l'éther et on lave plusieurs fois avec une solution de bicarbonate de sodium puis une solution saturée de chlorure de sodium.

La phase organique est séchée sur sulfate de magnésium et concentrée. Le liquide orange résiduel est chromatographié sur colonne (Phase : Florisil φ 0,075 - 0,150 mm - Eluant : gradient : pentane-10 éther-1 ---- éther).

On obtient 3,149 g d'un liquide jaune pâle identifié en RMN comme étant l'aldéhyde acétal attendu (mélange de 4 isomères). Rendement : 56 %.

| **RMN** : 1H RMN AM 360 (CDCl₃ - Réf. HMDS) | | |
|---|---|---|
| δ (ppm) | H⁻ | Attribution |
| 1,29 | 1 H | H₂' équatorial |
| 1,7 à 2,8 | 11 H | H'₂ axial 2H₅-2H₄-6H (méthyl) |
| 3,77 | 2 H | H'₁ axial |
| 4,07 | 2 H | H₁' équatorial |
| 5,25 | 1 H | H2 |
| 5,29 | | |
| | | |
| 6,42 | 1 H | H6 |
| 6,47 | | |

| δ H₈ | δ H₁ | ISOMERE |
|---|---|---|
| 10,08 | 5,13 | 2E,6Z |
| 10,05 | 5,07 | 2Z,6Z |
| 9,34 | 5,08 | 2Z,6E |
| 9,32 | 5,14 | 2E,6E |

### EXEMPLE 5

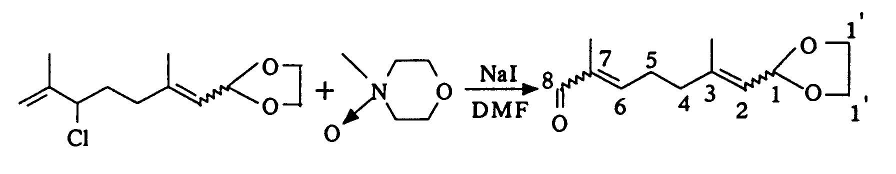

En opérant d'une façon analogue à celle de l'exemple 4, on obtient le mélange des 4 isomères de l'aldéhyde acétal C₁₀ caractérisé par analyse RMN.

| **RMN** : 1H 360 MHz (solvant CDCl₃ - Réf. HMDS) | | |
|---|---|---|
| δ (ppm) | H⁻ | Attribution |
| 1,6 à 2,6 | 10 H | 2H₄ - 2H₅ - 6H (méthy) |
| 3,8 à 4,1 | 4 H | 2 x 2 |
| 5,28 | | |
| 5,32 | 1 H | 1H2 (cis et trans) |
| environ 5,4 à 5,5 | 1 H | 1H₁ (cis et trans) |
| 9,38 | | |
| 9,39 | | H8 trans (isomères cis-trans acétal) |
| | | |
| 9,45 | 1 H | |
| | | |
| 9,46 | | H8 cis (isomères cis-trans acétal) |

### EXEMPLE 6

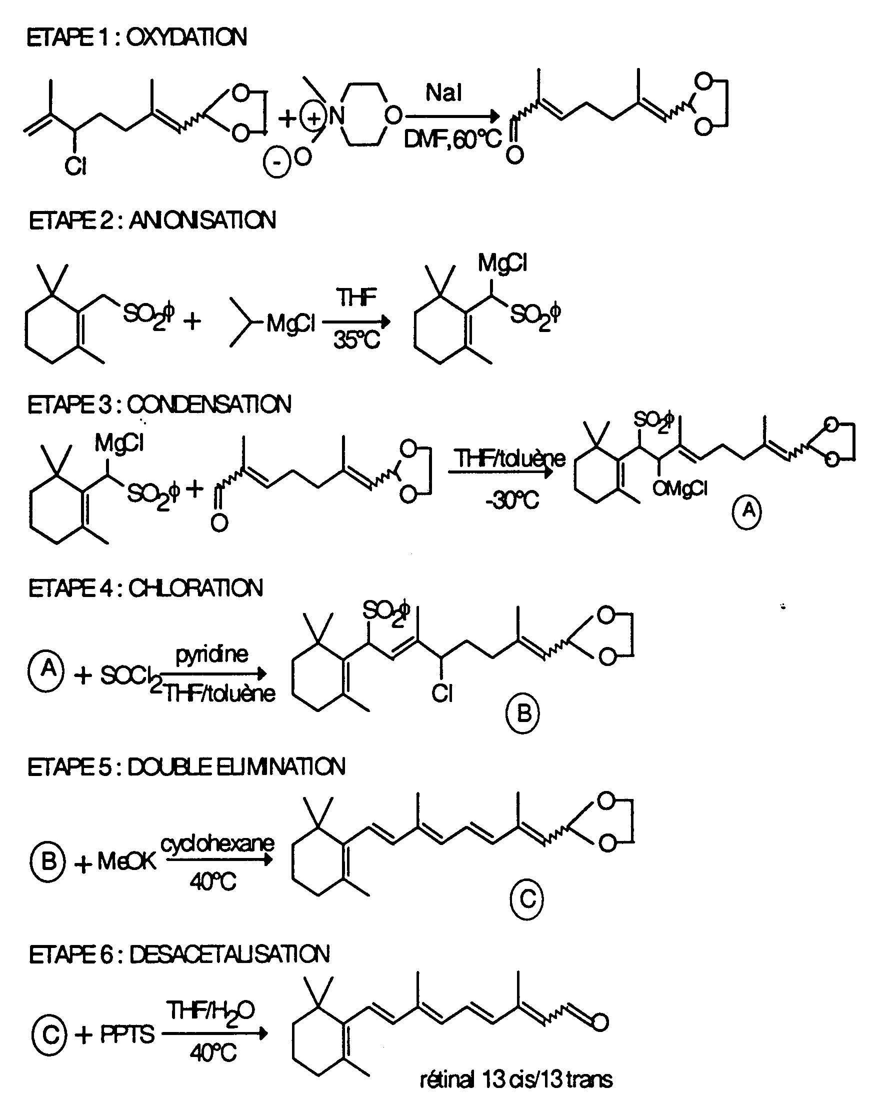

### ETAPE 1

Dans un bicol de 25 ml, muni d'un thermomètre, d'une agitation et balayé à l'argon, on charge :
- 0,779 g de 4-méthylmorpholine N-oxyde anhydre à 97 %
- 0,450 g d'iodure de sodium
- 0,585 g de chloroacétal glycolique
- 5,5 ml de DMF anhydre.

On chauffe à 60°C pendant 4 heures 30 puis on refroidit dans un bain de glace, on extrait à l'éther et on lave avec successivement des solutions saturées de bicarbonate de sodium et de chlorure de sodium (pH = 7).

On ajoute 4 ml de toluène dans la phase organique et on évapore l'éther.

### ETAPE 2

Dans un bicol de 50 ml muni d'une agitation, d'un thermomètre, d'une ampoule de coulée de 10 ml et balayé à l'argon, on charge :
- 0,825 g de cyclogéranylphényl sulfone à 95 %
- 7 ml de THF anhydre
- 1,35 ml d'une solution de chlorure d'isopropyle magnésium 2M dans le THF.

On agite 3 heures 30 à 35°C.

### ETAPE 3

Le tricol de l'étape 2 est plongé dans un bain à -30/-35°C. On charge la solution de l'aldéhyde acétal glycolique dans le toluène (cf. étape 1) dans l'ampoule de coulée. On coule en 7 mn cet aldéhyde et on rince l'ampoule avec 1,5 ml de toluène. On agite pendant 1 heure 45 à -30°C.

### ETAPE 4

On ajoute 0,540 ml de pyridine anhydre puis on coule 0,250 ml de chlorure de thionyle. On laisse réagir à -30°C pendant 45 mn puis 45 mn à 0°C.

On verse 20 ml d'eau glacée dans le milieu réactionnel. On extrait au toluène et on lave avec une solution saturée de bicarbonate de sodium (pH 8/9) puis une solution de chlorure de sodium (pH = 6/7).

La phase organique est séchée sur MgSO₄.

Après évaporation du toluène, on obtient 1,534 g d'un liquide orange trouble purifié par chromatographie (phase : Florisil φ 0,075 - 0,150 mm - Eluant AcOEt 20/Pentane 80 %). On obtient 0,597 g d'un mélange de sulfones chlorées C₂₀ acétals et aldéhydes. Rendement de l'ordre de 45 à 50 %.

### ETAPE 5

Dans un ballon de 25 ml muni d'une agitation et balayé à l'argon, on charge :
- 0,560 g du mélange obtenu à l'étape 4
- 15 ml de cyclohexane
- 0,838 g de méthylate de potassium à 95 %.

On agite pendant 3 heures à 40°C puis on hydrolyse avec une solution saturée de chlorure d'ammonium à 25°C. On extrait à l'éther isopropylique. On lave avec une solution saturée de chlorure de sodium (pH à 6 - 7).

Après séchage sur MgSO₄ et élimination du solvant, on obtient 0,362 g d'une huile rouge vif.

Un dosage RMN avec étalon interne (p-diméthoxybenzène donne la répartition suivante :
. acétal du rétinal 13 cis + 13 trans : 34 % (massique)
. rétinal 13 cis + 13 trans : 10 %

Rendement : 21 % (calculé sur le chloroacétal C₁₀

### ETAPE 6

Dans un ballon de 50 ml muni d'une agitation, on charge :
- 0,289 g du produit brut issu de l'étape 5
- 5 ml de THF
- 0,9 ml d'eau
- 0,012 g de p-toluènesulfonate de pyridinium.

On agite pendant 1 heure 30 à température ambiante et à l'abri de la lumière. Après extractions à l'éther, lavages avec des solutions saturées de NaHCO₃ et NaCl, séchage sur MgSO₄ et évaporation du solvant, on obtient 0,266 g d'une huile rouge que l'on purifie par chromatographie (phase : silice - Eluant : pentane-10 éther -1). On obtient 0,100 g de rétinal (78 % isomère tout-trans - 22 % isomère 13 cis). Rendement : 18 % (calculé sur le chloroacétal C₁₀).

### EXEMPLE 7

Les étapes 2 à 6 sont enchainées, sans isoler aucun intermédiaire.

### ETAPES 2 à 4

Dans un tricol de 50 ml, muni d'une ampoule de coulée de 10 ml, d'une agitation et balayé à l'argon, on charge :
- 0,81 g de cyclogéranylphényl sulfone à 95 %
- 7 ml de THF anhydre
- 1,4 ml d'une solution 2M de chlorure d'isopropylmagnésium dans le THF.

On agite pendant 1 heure 10 à 35°C. On refroidit à -30°C et on charge dans l'ampoule de coulée 0,627 g d'aldéhyde acétal obtenu selon l'étape 1 de l'exemple 6 à 95 % en solution dans 6 ml de toluène anhydre.

Cette solution est introduite dans le milieu réactionnel en 10 minutes. On laisse agiter 2 heures à -30°C puis on ajoute 0,560 ml de pyridine (en une seule fois). On charge alors goutte à goutte 0,260 ml de chlorure de thionyle. On laisse réagir 1 heure à -30°C et 1 heure à 0/5°C. On traite la masse réactionnelle par extractions au toluène et lavages avec des solutions saturées de bicarbonate de sodium et de chlorure de sodium.

La phase organique est concentrée. On obtient une huile orange que l'on place dans un ballon de 10 ml avec 40 ml de cyclohexane.

### ETAPE 5

On purge le ballon à l'argon, on charge 1,964 g de méthylate de potassium et on agite à 40°C pendant 3 heures.

La masse réactionnelle est extraite à l'éther isopropylique et lavée successivement avec des solutions de chlorure d'ammonium et de chlorure de sodium.

La phase organique est concentrée dans un ballon de 50 ml.

### ETAPE 6

Dans le ballon précédent, on ajoute :
- 15 ml de THF
- 2 ml d'eau
- 40 mg de p-toluène sulfonate de pyridinium.

On laisse agiter pendant 1 heure 30 à température ambiante. On extrait à l'éther. On lave plusieurs fois avec des solutions saturées de bicarbonate de sodium et de chlorure de sodium (pH = 6-7).

La phase organique est séchée sur sulfate de magnésium et concentrée. On obtient 1,054 g d'une huile jaune-orange que l'on purifie sur colonne de silice (Phase : silice amicon φ 0,020 - 0,045 mm - Eluant : éther-1 pentane-7).

Le produit récupéré contient 0,394 g de rétinal, soit un rendement (par rapport à l'aldéhyde acétal) de 52 % réparti en :

| | |
|---|---|
| - tout trans | 46 % |
| - 13 cis | 49 % |
| - autre | <5% |

La double liaison 2,3 de l'acétal C₁₀ de départ était :

| | |
|---|---|
| - trans | 48 % |
| - cis | 52 % |

### ETAPE 7

On reprend le mélange d'isomères précédent que l'on dissous dans 2 ml d'éther éthylique. On ajoute 100 mg d'hydroquinone. Après agitation on ajoute 0,1 ml d'acide iodhydrique. On laisse reposer une nuit et on filtre et on obtient 0,33 g de complexe.

On introduit le complexe obtenu dans 2 ml d'une solution méthanol-eau (90/10) dans laquelle on ajoute 10 mg de borohydrure de sodium. Le rétinol obtenu (340 mg) est analysé par chromatographie liquide. Il présente la répartition suivante :

| | |
|---|---|
| - tout trans | 95,5 % |
| - 13 cis | 4 % |
| - 9 cis | 0,5 % |

## Revendications

1. Intermédiaire de préparation dans la synthèse de la vitamine A répondant à la formule (I) : 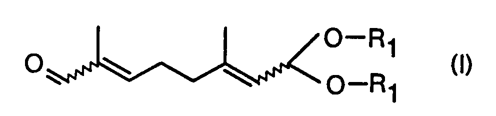 dans laquelle R₁ représente un groupe alkyle contenant 1 à 6 atomes de carbone ou un groupe alkylidène contenant 2 à 6 atomes de carbone.

2. Intermédiaire de préparation du composé de formule (I) répondant à la formule (II) : 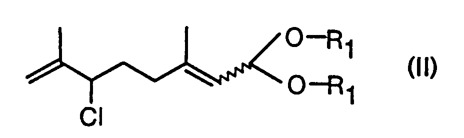 dans laquelle R₁ à la même signification que dans la formule (I).

3. Procédé de préparation de l'intermédiaire de formule (I) 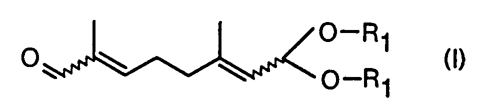 caractérisé en ce que l'on met en présence le composé de formule (II) 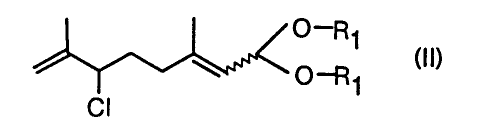 avec un N-oxyde d'amine tertiaire.

4. Procédé de préparation de l'intermédiaire de formule (II) 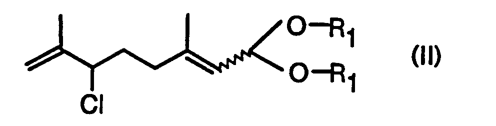 caractérisé en ce que l'on réalise une chloration du citral puis une acétalisation du chlorocitral obtenu.

5. Procédé de préparation de l'intermédiaire de formule (II) 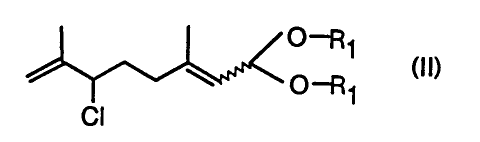 caractérisé en ce que l'on réalise une acétalisation du citral puis une chloration de l'acétal obtenu.

6. Procédé de préparation de la vitamine A caractérisé en ce que :
- dans une première étape, on condense la cyclogéranylsulfone sur le composé de formule (I) 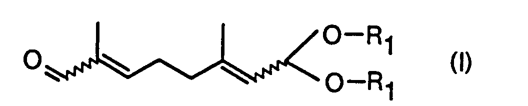
- dans une deuxième étape, on réalise une halogénation du composé obtenu à l'étape 1,
- dans une troisième étape, on réalise une double élimination du groupe sulfone et de l'halogène,
- dans une quatrième étape, on élimine le groupe acétal,
- dans une cinqième étape, on réalise le redressement du rétinal obtenu à l'étape 4.

7. Procédé selon la revendication 6 caractérisé en ce que la première étape est réalisée dans un solvant hydrocarboné en présence d'une base.

8. Procédé selon la revendication 6 caractérisé en ce que deuxième étape est réalisée en présence de chlorure de thionyle ou de trichlorure de phosphore et en présence d'une amine tertiaire.

9. Procédé selon la revendication 6 caractérisé en ce que la troisième étape est réalisée en présence d'un alcoolate alcalin dans un hydrocarbure.

10. Procédé selon la revendication 6 caractérisé en ce que que la quatrième étape est réalisée en présence d'un ammonium quaternaire.

11. Procédé selon la revendication 6 caractérisé en ce que la cinquième étape est réalisée en présence de dihydroxybenzènze.

## Claims

1. Preparation intermediate in the synthesis of vitamin A corresponding to the formula (I): 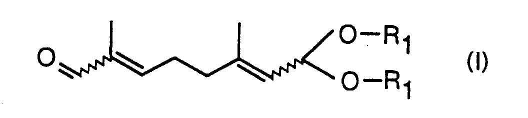 in which R₁ represents an alkyl group containing 1 to 6 carbon atoms or an alkylidene group containing 2 to 6 carbon atoms.

2. Intermediate in the preparation of the compound of formula (I) corresponding to the formula (II): 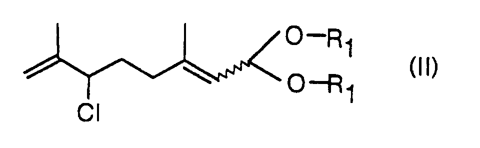 in which R₁ has the same meaning as in the formula (I).

3. Process for the preparation of the intermediate of formula (I) 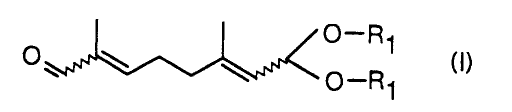 characterized in that the compound of formula (II) 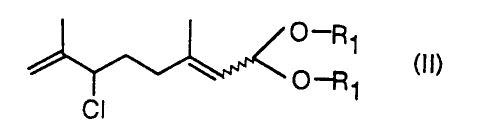 is brought into contact with an N-oxide of a tertiary amine.

4. Process for the preparation of the intermediate of formula (II) 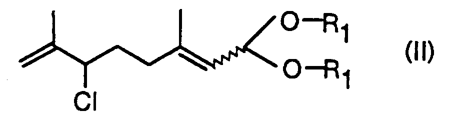 characterized in that citral is chlorinated and then the chlorocitral obtained is acetalised.

5. Process for the preparation of the intermediate of formula (II) 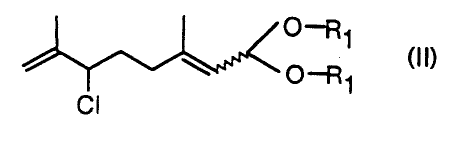 characterized in that citral is acetalised and then the acetal obtained is chlorinated.

6. Process for the preparation of vitamin A, characterized in that:
- in a first stage, cyclogeranyl sulphone is condensed with the compound of formula (I) 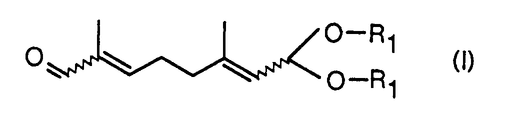
- in a second stage, the compound obtained in Stage 1 is halogenated,
- in a third stage, a double removal of the sulphone group and of the halogen is carried out,
- in a fourth stage, the acetal group is removed,
- in a fifth stage, the retinal obtained in Stage 4 is isomerized to the desired configuration.

7. Process according to claim 6, characterized in that the first stage is carried out in a hydrocarbon solvent in the presence of a base.

8. Process according to claim 6, characterized in that the second stage is carried out in the presence of thionyl chloride or of phosphorus trichloride and in the presence of a tertiary amine.

9. Process according to claim 6, characterized in that the third stage is carried out in the presence of an alkali metal alkoxide in a hydrocarbon.

10. Process according to claim 6, characterized in that the fourth stage is carried out in the presence of a quaternary ammonium.

11. Process according to claim 6, characterized in that the fifth stage is carried out in the presence of dihydroxybenzene.

## Patentansprüche

1. Zwischenprodukt zur Herstellung bei der Synthese von Vitamin A, das der Formel (I) 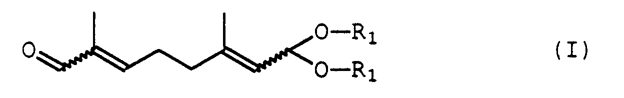 entspricht, in der R₁ eine Alkylgruppe mit 1 bis 6 Kohlenstoffatomen oder eine Alkylidengruppe mit 2 bis 6 Kohlenstoffatomen darstellt.

2. Zwischenprodukt zur Herstellung der Verbindung der Formel (I), das der Formel (II) 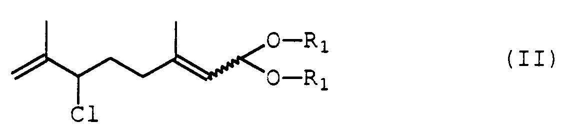 entspricht, in der R₁ die gleiche Bedeutung wie in Formel (I) besitzt.

3. Verfahren zur Herstellung des Zwischenproduktes der Formel (I) 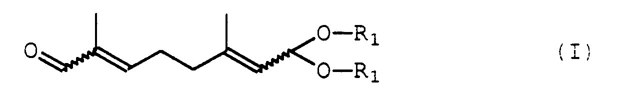 dadurch gekennzeichnet, daß man die Verbindung der Formel (II) 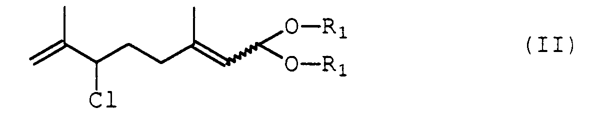 mit einem tertiären Amin-N-oxid zusammenbringt.

4. Verfahren zur Herstellung des Zwischenproduktes der Formel (II) 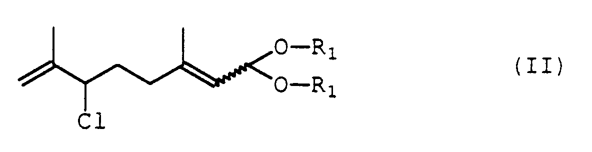 dadurch gekennzeichnet, daß man eine Chlorierung von Citral durchfuhrt, gefolgt von einer Acetalisierung des erhaltenen Chlorcitrals.

5. Verfahren zur Herstellung des Zwischenproduktes der Formel (II) 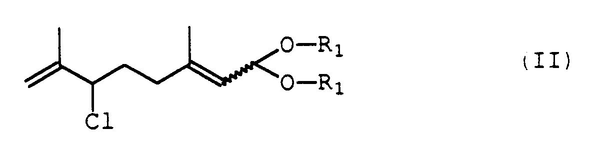 dadurch gekennzeichnet, daß man eine Acetalisierung von Citral durchführt, gefolgt von einer Chlorierung des erhaltenen Acetals.

6. Verfahren zur Herstellung von Vitamin A, dadurch gekennzeichnet, daß man
- in einer ersten Stufe Cyclogeranylsulfon mit der Verbindung der Formel (I) 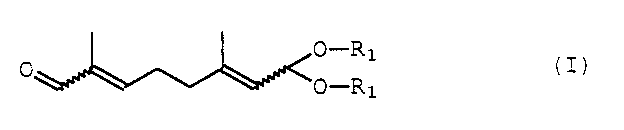 kondensiert,
- in einer zweiten Stufe eine Halogenierung der in Stufe 1 erhaltenen Verbindung durchführt,
- in einer dritten Stufe eine doppelte Entfernung der Sulfongruppe und des Halogens durchführt,
- in einer vierten Stufe die Acetalgruppe entfernt,
- in einer fünften Stufe die Aufrichtung des in Stufe 4 erhaltenen Retinals vornimmt.

7. Verfahren nach Anspruch 6, dadurch gekennzeichnet, daß die erste Stufe in einem Kohlenwasserstoff-Lösungsmittel in Anwesenheit einer Base durchgeführt wird.

8. Verfahren nach Anspruch 6, dadurch gekennzeichnet, daß die zweite Stufe in Anwesenheit von Thionylchlorid oder Phosphortrichlorid und in Anwesenheit eines tertiären Amins durchgeführt wird.

9. Verfahren nach Anspruch 6, dadurch gekennzeichnet, daß die dritte Stufe in Anwesenheit eines Alkalialkoholates in einem Kohlenwasserstoff durchgeführt wird.

10. Verfahren nach Anspruch 6, dadurch gekennzeichnet, daß die vierte Stufe in Anwesenheit eines quaternären Ammoniums durchgeführt wird.

11. Verfahren nach Anspruch 6, dadurch gekennzeichnet, daß die fünfte Stufe in Anwesenheit von Dihydroxybenzol durchgeführt wird.
